# EUROPEAN PATENT SPECIFICATION

(11) **EP 3 517 056 B1**
(45) Date of publication and mention of the grant of the patent: **11.11.2020**
(21) Application number: 19153231.6
(22) Date of filing: 23.01.2019
(51) Int. Cl.: A61B 17/122, A61B 17/00

(54) **LIGATION CLIP WITH TISSUE RENTENTION FEATURES**
LIGATURCLIP MIT GEWEBEHALTEFUNKTION
PINCE DE LIGATURE AVEC DES CARACTÉRISTIQUES DE RÉTENTION DE TISSUS

(30) Priority: 24.01.2018 US 201862621163 P; 12.10.2018 US 201816158368
(43) Date of publication of application: 31.07.2019
(73) Proprietor: Covidien LP, Mansfield, MA 02048 (US)
(72) Inventor: PILLETERE, Roy, North Haven, CT 06473 (US); BANERJEE, Saumya, Hamden, CT 06514 (US); THOMAS, Justin, New Haven, CT 06512 (US); DININO, Matthew, Newington, CT 06111 (US); BROWN, Eric, Haddam, CT 06438 (US); MORCK, Gregory, Middletown, CT 06457 (US)
(74) Representative: Maschio, Antonio

(56) References cited:
- WO-A1-2013/059826
- WO-A1-2016/205343
- US-A- 5 921 991
- US-B1- 9 282 972

## Description

### CROSS-REFERENCE TO RELATED APPLICATION

This application claims the benefit of and priority to U.S. Provisional Patent Application No. 62/621,163 filed January 24, 2018.

### BACKGROUND

### 1. Technical Description

The present disclosure is directed to ligation clips for sealing body vessels and, more particularly, to ligation clips that include clamping surfaces with tissue retention features for sealing body vessels.

### 2. Background of Related Art

Ligation clips are well known in the surgical arts and are commonly used during a variety of surgical procedures to ligate tissue, e.g., a body vessel. Typically, ligation clips include first and second jaws that include clamping surfaces. The jaws are pivotably connected to each other and movable between open and clamped positions. In use, the ligation clip is clamped to a portion of the body vessel to clamp the body vessel between the clamping surfaces of the first and second jaws. After the ligation clip is clamped about the body vessel, any movement of the ligation clip in relation to the body vessel may have a negative impact on the performance of the ligation clip. This may affect the success of the surgical procedure and threaten the well-being of the patient.

United States Patent US 9,282,972 B1 discloses a surgical clip including a pair of opposed arms joined at one end by an integrally formed flexible hinge and a hinge lock. The clamping side of an arm includes a protruding feature, such as a wedge shape elongated ridge, while the clamping side of the opposite arms includes a corresponding trough or aperture that receives the protruding feature. Clamping surfaces may include non-slip protrusions, such as ribs, ridges, cones or pins.

International Patent Application WO 2016/205343 A1 discloses a surgical clip including a first leg member including a first inner surface and a first plurality of protrusions disposed on the first inner surfaces and a second leg member including a second inner surface and a second plurality of protrusions disposed on the second inner surface. The first and second plurality of protrusions includes a gable structure that extends along a longitudinal direction of the first or second inner surface. The orientation and the geometric shape of the protrusions of the surgical clip allow for increased resistance to the migration or sliding of the clip along a longitudinal direction of the blood vessel or tissue structure, while providing a balanced closure force. The surgical clip can prevent the longitudinal migration along the blood vessel or tissue structure.

International Patent Application WO 2013/059826 A1 discloses a surgical ligation clip including a top jaw member and bottom jaw member joined at a hinge section for movement about the hinge section. The hinge section can be offset or space laterally from one or both of the jaw members.

Teeth arrangements and distributions are also described, as well as closing and jaw ends engagement configurations, for example for more easily engaging/piercing connective or other tissue.

United States Patent US 5,921,991 discloses umbilical cord clamps made of a flexible material. The clamps include a pair of elongated arms of the clamps. The arms are flexibly connected to each other via a flexible hinge and include serrated surfaces on their inner sides.

A continuing need exists in the art for ligation clips that have improved retention characteristics to prevent movement of the ligation clip in relation to a body vessel after placement.

### SUMMARY

The present invention is defined by the features of the independent claim. Preferred embodiments are given in the dependent claims.

### BRIEF DESCRIPTION OF THE DRAWINGS

Various exemplary embodiments of the presently disclosed ligation clip are described herein below with reference to the drawings, wherein:
FIG. 1 is a side perspective view of an exemplary embodiment of the presently disclosed ligation clip in an open position;
FIG. 2 is an enlarged view of the indicated area of detail shown in FIG. 1;
FIG. 2A is an enlarged top view of a portion of a jaw of the ligation clip shown in FIG. 1;
FIG. 3 is a side perspective view of the ligation clip shown in FIG.1 in the clamped position;
FIG. 4 is an enlarged view of the indicated area of detail shown in FIG. 3;
FIG. 5 is a side view of the ligation clip shown in FIG. 1 in the open position placed about tissue;
FIG. 6 is a side view of the ligation clip shown in FIG. 5 in the clamped position placed about tissue; and
FIG. 7 is an enlarged view of the indicated area of detail shown in FIG. 6.

### DETAILED DESCRIPTION OF EMBODIMENTS

The presently disclosed ligation clip will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel. Further, the term "about" is used generally to mean plus or minus 10 percent of the disclosed parameter.

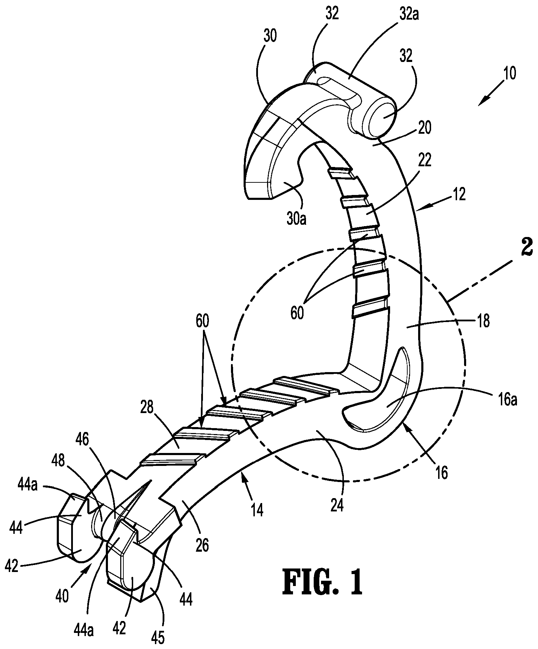

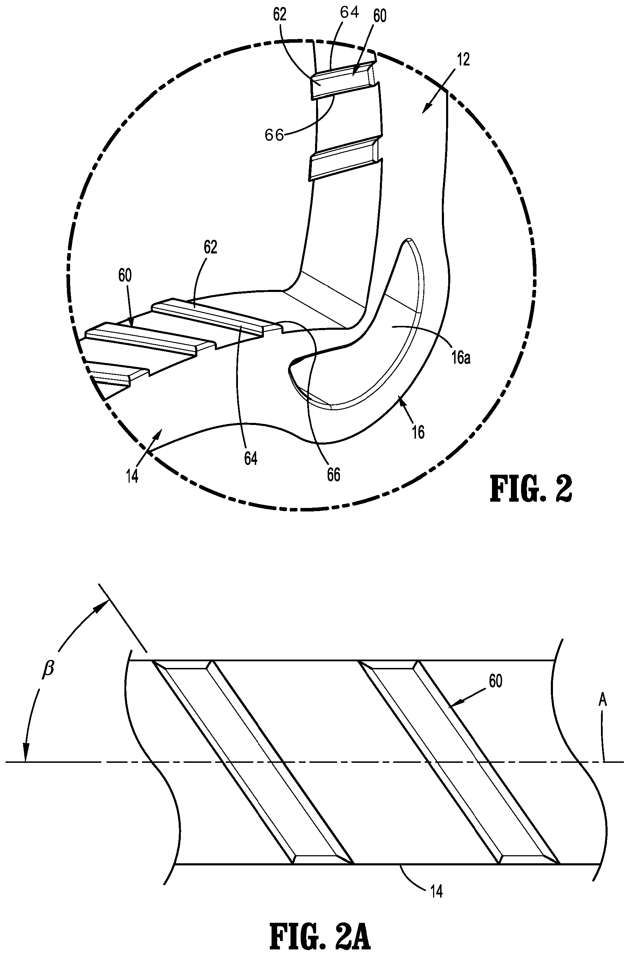

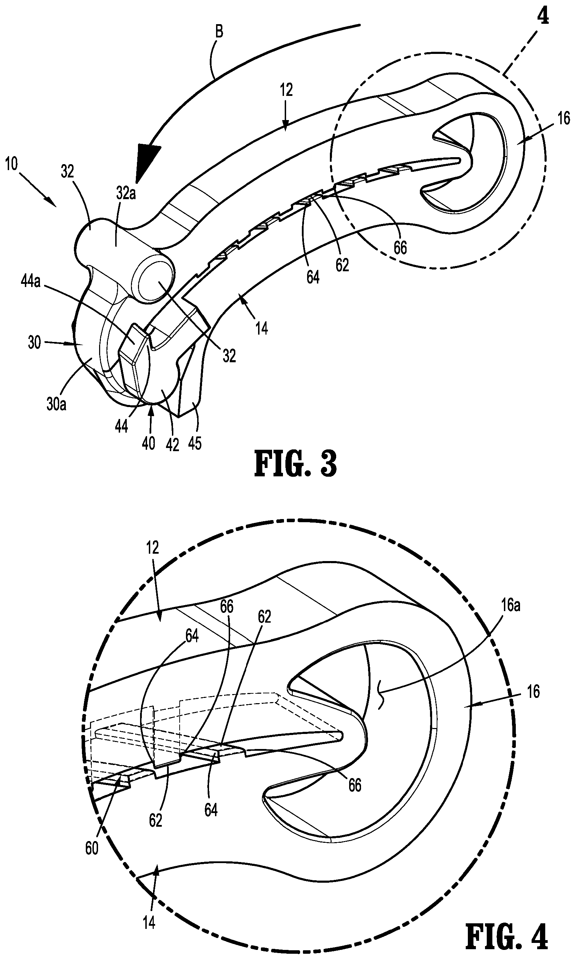

Referring to FIGS. 1-4, an exemplary embodiment of the presently disclosed ligation clip is shown generally as 10. The ligation clip 10 defines a longitudinal axis "A" (FIG. 2A) and includes a first jaw 12, a second jaw 14, and a hinge portion 16 coupling the first jaw 12 to the second jaw 14. The first jaw 12 is pivotable in relation to the second jaw 14 about the hinge portion 16 to move the ligation clip 10 between an open position (FIG. 1) and a clamped position (FIG. 3). In embodiments, the first and second jaws 12, 14 are curved along the longitudinal axis "A" of the ligation clip 10 although other jaw configurations are envisioned. The hinge portion 16 can be integrally formed with the first and second jaws 12, 14, e.g., a living hinge, and may define a crescent shaped through bore 16a to facilitate movement of the first jaw 12 in relation to the second jaw 14 between the open and clamped positions. The through bore 16a also allows for substantially complete closure of the first and second jaws 12, 14 adjacent a proximal end of the jaws 12, 14.

The first jaw 12 defines a longitudinal axis and includes a proximal portion 18, a distal portion 20, and a clamping surface 22. The second jaw 14 defines a longitudinal axis that is parallel to the longitudinal axis of the first jaw 12 and includes a proximal portion 24, a distal portion 26, and a clamping surface 28. The proximal portions 18, 24, of the first and second jaws 12, 14, respectively, are coupled to the hinge portion 16.

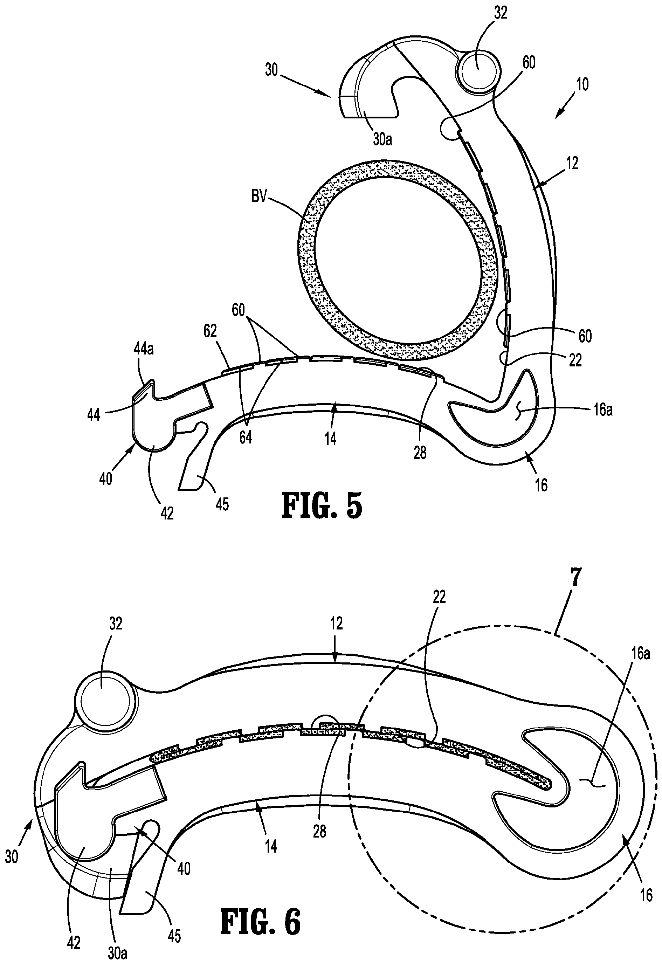

The distal portion 20 of first jaw 12 includes a first locking element 30 and spaced bosses 32. The first locking element 30 has a hooked portion 30a that extends downwardly and proximally from the tissue clamping surface 22 to define a portion of a latching mechanism (FIG. 6) that is configured to retain the ligation clip 10 in a clamped position as described in further detail below. The bosses 32 are positioned and configured to engage the jaws of an applicator (not shown) to facilitate application of the ligation clip 10 to tissue. In embodiments, the bosses 32 may be form a portion of a single cylindrical member 32a.

The distal portion 26 of the second jaw 14 includes a second locking element 40, spaced bosses 42, tissue engaging members 44, and a resilient retaining member 45. The resilient retaining member 45 is positioned to be engaged by the first locking element 30 of the first jaw 12 when the ligation clip 10 is moved to the clamped position such that the retaining member is biased into the first locking element 30 to urge the first locking element 30 in relation to the second locking element 40 to the latched position (FIG. 6). The second locking element 40 defines a recess 46 and a cam surface 48. The cam surface 48 and the recess 46 are configured to receive and guide the first locking element 30 into locking engagement with the second locking element 40 when the ligation clip 10 is moved to the clamped position (FIG. 3) to secure the ligation clip 10 in the clamped position. In particular, the cam surface 48 is configured to deflect the first locking element 30 outwardly in a distal direction as the first locking element 30 passes over the cam surface 48 such that when the first locking element 30 moves past the cam surface 48, the first locking element 30 resiliently moves inwardly into engagement with the second locking element 40 to retain the ligation clip 10 in the clamped position.

In embodiments, each of the tissue engaging members 44 has a downwardly sloped distal face 44a that is configured to grip and stretch tissue as the ligation clip 10 is moved from the open position (FIG. 1) towards the clamped position (FIG. 3) to improve the ligating characteristics of the ligation clip 10. The bosses 42 are similar to the bosses 32 of the first jaw 12 and are configured to engage jaws of an applicator (not shown) to facilitate application of the ligation clip 10 to tissue.

The first and second clamping surfaces 22, 28 of the first and second jaws 12, 14, respectively, are substantially flat and support a plurality of diagonal ribs 60. Each of the ribs 60 defines an angle β (FIG. 2A) with the longitudinal axis "A" of the ligation clip 10. In exemplary ligation clips, the angle β is between about 60 degrees and about 90 degrees. According to the present invention, the angle β is between about 75 degrees and about 85 degrees. In embodiments, the angle β is between about 80 degrees.

In embodiments, each of the ribs 60 has a substantially rectangular configuration including a wall 62 in opposition to the tissue clamping surface 22, 28 of the respective opposing jaw 12, 14, and substantially vertical distal and proximal walls 64 and 66, respectively. In embodiments, the opposing wall 62 is substantially flat and the distal and proximal walls 64, 66 have a small degree of slope. Alternately, the proximal and distal walls can be vertical or include more of a slope and the opposing wall 62 need not be flat but rather may be curved or include tissue engaging structure such as ribs, knurls or the like. In some embodiments, each or some of the ribs 60 extend entirely across the clamping surface 22, 28 of the respective first or second jaw 12, 14. Alternately, some or all of the ribs 60 on one or both of the clamping surfaces 22, 28 may only extend partly across the clamping surface 22, 28 of the respective first or second jaw 12, 14.

Each of the ribs 60 of the first jaw 12 includes a longitudinal axis that is substantially parallel to the longitudinal axis of the ribs 60 supported on the second jaw 14. The size and axial spacing of the ribs 60 on each of the clamping surfaces 22, 28 of the first and second jaws 28 may vary.

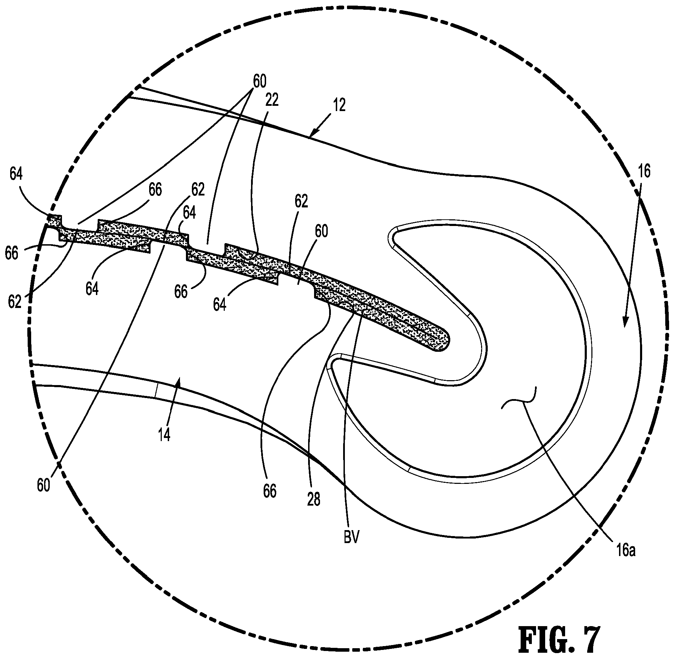

In embodiments, the distal wall 64 of the ribs 60 on the first jaw 12 is positioned to be in substantial alignment with the proximal wall 66 of the ribs 60 on the second jaw 14 when the ligation clip 10 is in the clamped position. As used herein, "substantial alignment" means that the distal wall 64 of the ribs 60 on the first jaw 12 are positioned in close proximity with a respective one of the proximal walls 66 of the ribs 60 on the second jaw 14 such that the distal wall 64 is positioned in full alignment, offset slightly distally, or offset slightly proximally than the proximal wall 66 of the ribs 60 of the second jaw 14. In some embodiments, the opposing walls 62 of the ribs 60 on opposing jaws 12, 14 are axially spaced and do not overlap when the ligation clip 10 is in the clamped position. With the ribs 60 positioned in this manner, a body vessel "BV" (FIG. 7) supported between the jaws 12, 14 is compressed between the opposing walls 62 of the ribs and the clamping surfaces 22, 28 of the first and second jaws 12, 14, and between the proximal and distal walls 62, 64 of the ribs 60 on the clamping surfaces 22, 28 of the first and second jaws 12, 14, respectively. This combined clamping action improves the retention capabilities of the ligation clip 10 on the body vessel "BV".

Referring to FIGS. 3 and 4, when the first jaw 12 and the second jaw 14 are moved from the open position (FIG. 1) to the clamped position (FIG. 3) in the direction indicated by arrow "B" in FIG. 3, the first jaw 12 pivots in relation to the second jaw 14 about the hinge portion 16 to move the hooked portion 30a of the first locking element 30 into engagement with the second locking element 40 to secure the ligation clip 10 in the clamped position. In the clamped position, the distal walls of the ribs 60 on the clamping surface 22 of the first jaw 12 are positioned in close alignment and proximity to the proximal walls of the ribs 60 formed on the second jaw 14 as described above.

In embodiments, the surgical ligation clip 10 may be comprised of a resilient bioabsorbable and/or biocompatible polymeric material. Examples of suitable bioabsorbable and/or biocompatible polymers include acetal polyoxymethylene (POM), polyethylene terephthalate (PET), polybutylene terephthalate (PBT), polyoxymethylene, polyetheretherketone (PEEK), polypropylene, and polyethylene or other thermoplastic materials having similar properties that can be injection-molded. The ligation clip 10 may also be comprised of a polymeric material or materials in combination with radiolucent metal alloys. Alternately, other materials may be used to form the ligation clip 10 including biocompatible metals, plastics and composites.

Referring to FIGS. 5-7, in use, the ligation clip 10 is positioned about tissue, e.g., a body vessel "BV", such that the body vessel "BV" is positioned between the tissue clamping surface 22 of the first jaw 12 and the tissue clamping surface 28 of the second jaw 14. As described above, when the ligation clip 10 is moved from the open position (FIG. 1) to the closed position (FIG. 6), the first jaw 12 pivots in relation to the second jaw 14 about the hinge 16 to move the hooked portion 30a of the first locking element 30 into engagement with the second locking element 40 to secure the ligation clip 10 in the clamped position.

As shown in FIGS. 6 and 7, in the clamped position, the body vessel "BV" is compressed between the first and second clamping surfaces 22, 28 of the first and second jaws 12, 14, respectively. More particularly, when the ligation clip 10 is moved to the clamped position, the body vessel "BV" is compressed between the clamping surfaces 22, 28 of the first and second jaws 12, 14, respectively. In addition, portions of the body vessel "BV" are compressed between the opposing walls 62 of the ribs 60 and the clamping surfaces 22, 28 of the first and second jaws 12, 14, respectively, and between the distal walls 64 of the ribs 60 of the first jaw 12 and the proximal walls 66 of the ribs 60 of the second jaw 14. As described above, this combination of compression forces improves retention of the ligation clip 10 about a body vessel "BV" and minimizes the likelihood that the ligation clip 10 will move in relation to the body vessel "BV" after placement of the ligation clip 10.

Persons skilled in the art will understand that the devices specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A ligation clip (10) defining a longitudinal axis (A) and comprising:
a first jaw (12) having a first clamping surface (22), the first clamping surface (22) supporting first ribs (60), each of the first ribs (60) being axially spaced from each other along the first clamping surface (22) and defining an axis that defines an angle β with the longitudinal axis (A) of the ligation clip (10); and
a second jaw (14) having a second clamping surface (28) in opposition to the first clamping surface (22), the second clamping surface (28) supporting second ribs (60), each of the second ribs (60) being spaced from each other along the second clamping surface (28) and defining an axis that defines the angle β with the longitudinal axis (A) of the ligation clip (10), the second jaw (14) being pivotably coupled to the first jaw (12) such that the ligation clip (10) is movable between an open position and a clamped position;
wherein each of the first ribs (60) includes a distal wall (64) and a proximal wall (66) and a wall (62) in opposition to the second clamping surface (28) and each of the second ribs (60) includes a distal wall (64), a proximal wall (66), and a wall (62) in opposition to the first clamping surface (22), the distal wall (64) of at least some of the first ribs (60) being positioned in substantial alignment with the proximal wall (66) of a respective one of the second ribs (60) when the ligation clip (10) is in the clamped position,
**characterised in that** the angle β is between about 75 degrees and about 85 degrees.

2. The ligation clip (10) of claim 1, wherein the angle β is about 80 degrees.

3. The ligation clip (10) of any preceding claim, wherein the first and second jaws (12, 14) support bosses (32, 42), the bosses (32, 42) being configured to engage a clip applier.

4. The ligation clip (10) of any preceding claim, wherein the ligation clip (10) is formed of a polymeric material.

5. The ligation clip (10) of any preceding claim, wherein the first jaw (12) includes a first locking element (30) and the second jaw (14) includes a second locking element (40), the second locking element (40) being pivotable into engagement with the first locking element (30) to retain the ligation clip (10) in the clamped position.

6. The ligation clip (10) of any preceding claim, wherein the first and second jaws (12, 14) are curved along the longitudinal axis of the ligation clip (10).

7. The ligation clip (10) of any preceding claim, wherein the second jaw (14) supports tissue engaging members (44) that are configured to grip and stretch tissue as the ligation clip (10) is moved from the open position towards the clamped position.

8. The ligation clip (10) of claim 7, wherein each of the tissue engaging members (44) includes a distal face (44a) that is downwardly sloped in a distal direction.

9. The ligation clip (10) of any preceding claim, wherein the first jaw (12) is pivotably coupled to the second jaw (14) by a living hinge.

10. The ligation clip (10) of any preceding claim, wherein the ribs (60) have a rectangular configuration.

11. The ligation clip (10) of any preceding claim, wherein the walls (62) of the first ribs (60) in opposition to the second clamping surface (28) of the second jaw (14) and the walls (62) of the second ribs (60) in opposition to the first clamping surface (22) of the first jaw (12) are flat; or wherein the walls (62) of the first ribs (60) in opposition to the second clamping surface (28) of the second jaw (14) and the walls (62) of the second ribs (60) in opposition to the first clamping surface (22) of the first jaw (12) are curved.

12. The ligation clip (10) of claim 5, wherein the second jaw (14) includes a resilient retaining member (45), the resilient retaining member (45) being positioned to be engaged by the first locking element (30) of the first jaw (12) when the ligation clip (10) is moved to the clamped position to retain the ligation clip (10) in the clamped position.

## Patentansprüche

1. Ligaturklammer (10), die eine Längsachse (A) definiert und Folgendes umfasst:
eine erste Backe (12) mit einer ersten Klemmfläche (22), wobei die erste Klemmfläche (22) die ersten Rippen (60) trägt, wobei jede der ersten Rippen (60) axial voneinander entlang der ersten Klemmfläche (22) beabstandet ist und eine Achse definiert, die einen Winkel β mit der Längsachse (A) der Ligaturklammer (10) definiert; und
eine zweite Backe (14) mit einer zweiten Klemmfläche (28), die der ersten Klemmfläche (22) gegenüberliegt, wobei die zweite Klemmfläche (28) zweite Rippen (60) trägt, wobei jede der zweiten Rippen (60) entlang der zweiten Klemmfläche (28) voneinander beabstandet ist und eine Achse definiert, die den Winkel β mit der Längsachse (A) der Ligaturklammer (10) definiert, wobei die zweite Backe (14) schwenkbar derart mit der ersten Backe (12) gekoppelt ist, dass die Ligaturklammer (10) zwischen einer offenen Position und einer Klemmposition bewegbar ist;
wobei jede der ersten Rippen (60) eine distale Wand (64) und eine proximale Wand (66) und eine der zweiten Klemmfläche (28) gegenüberliegende Wand (62) aufweist und jede der zweiten Rippen (60) eine distale Wand (64), eine proximale Wand (66) und eine der ersten Klemmfläche (22) gegenüberliegende Wand (62) aufweist, wobei die distale Wand (64) mindestens einige der ersten Rippen (60) im Wesentlichen mit der proximalen Wand (66) einer jeweiligen der zweiten Rippen (60) ausgerichtet ist, wenn sich die Ligaturklammer (10) in der Klemmstellung befindet, **dadurch gekennzeichnet, dass** der Winkel β zwischen etwa 75 Grad und etwa 85 Grad liegt.

2. Ligaturklammer (10) nach Anspruch 1, wobei der Winkel β etwa 80 Grad beträgt.

3. Ligaturklammer (10) nach einem der vorhergehenden Ansprüche, wobei die erste und zweite Backe (12, 14) Vorsprünge (32, 42) tragen, wobei die Vorsprünge (32, 42) so konfiguriert sind, dass sie in eine Klammer-Anbringvorrichtung eingreifen.

4. Ligaturklammer (10) nach einem der vorhergehenden Ansprüche, wobei die Ligaturklammer (10) aus einem polymeren Material gebildet ist.

5. Ligaturklammer (10) nach einem der vorhergehenden Ansprüche, wobei die erste Backe (12) ein erstes Verriegelungselement (30) und die zweite Backe (14) ein zweites Verriegelungselement (40) aufweist, wobei das zweite Verriegelungselement (40) in Eingriff mit dem ersten Verriegelungselement (30) schwenkbar ist, um die Ligaturklammer (10) in der geklemmten Position zu halten.

6. Ligaturklammer (10) nach einem der vorhergehenden Ansprüche, wobei die erste und zweite Backe (12, 14) entlang der Längsachse der Ligaturklammer (10) gekrümmt sind.

7. Ligaturklammer (10) nach einem der vorhergehenden Ansprüche, wobei die zweite Backe (14) Gewebeeingriffselemente (44) trägt, die so konfiguriert sind, dass sie Gewebe greifen und dehnen, wenn die Ligaturklammer (10) von der offenen Position in die geklemmte Position bewegt wird.

8. Ligaturklammer (10) nach Anspruch 7, wobei jedes der Gewebeeingriffselemente (44) eine distale Fläche (44a) aufweist, die in einer distalen Richtung nach unten geneigt ist.

9. Ligaturklammer (10) nach einem der vorhergehenden Ansprüche, wobei die erste Backe (12) durch ein lebendes Scharnier schwenkbar mit der zweiten Backe (14) verbunden ist.

10. Ligaturklammer (10) nach einem der vorhergehenden Ansprüche, wobei die Rippen (60) eine rechteckige Konfiguration aufweisen.

11. Ligaturklammer (10) nach einem der vorhergehenden Ansprüche, wobei die Wände (62) der ersten Rippen (60) gegenüber der zweiten Klemmfläche (28) der zweiten Backe (14) und die Wände (62) der zweiten Rippen (60) gegenüber der ersten Klemmfläche (22) der ersten Backe (12) flach sind; oder
wobei die Wände (62) der ersten Rippen (60) gegenüber der zweiten Klemmfläche (28) der zweiten Backe (14) und die Wände (62) der zweiten Rippen (60) gegenüber der ersten Klemmfläche (22) der ersten Backe (12) gekrümmt sind.

12. Ligaturklammer (10) nach Anspruch 5, wobei die zweite Backe (14) ein elastisches Halteelement (45) aufweist, wobei das elastische Halteelement (45) so positioniert ist, dass es mit dem ersten Verriegelungselement (30) der ersten Backe (12) in Eingriff kommt, wenn die Ligaturklammer (10) in die geklemmte Position bewegt wird, um die Ligaturklammer (10) in der geklemmten Position zu halten.

## Revendications

1. Pince de ligature (10) définissant un axe longitudinal (A) et comprenant :
une première mâchoire (12) présentant une première surface de pincement (22), la première surface de pincement (22) soutenant des premières nervures (60), chacune des premières nervures (60) étant axialement espacées l'une de l'autre le long de la première surface de pincement (22) et définissant un axe qui définit un angle β avec l'axe longitudinal (A) de la pince de ligature (10) ; et
une seconde mâchoire (14) présentant une seconde surface de pincement (28) en opposition à la première surface de pincement (22), la seconde surface de pincement (28) soutenant des secondes nervures (60), chacune des secondes nervures (60) étant espacée l'une de l'autre le long de la seconde surface de pincement (28) et définissant un axe qui définit l'angle β avec l'axe longitudinal (A) de la pince de ligature (10), la seconde mâchoire (14) étant accouplée de manière pivotante à la première mâchoire (12) de sorte que la pince de ligature (10) soit mobile entre une position ouverte et une position pincée ;
chacune des premières nervures (60) comportant une paroi distale (64), une paroi proximale (66) et une paroi (62) en opposition à la seconde surface de pincement (28) et chacune des secondes nervures (60) comportant un paroi distale (64), une paroi proximale (66) et une paroi (62) en opposition à la première surface de pincement (22), la paroi distale (64) d'au moins certaines des premières nervures (60) étant positionnée en alignement sensible avec la paroi proximale (66) de l'une respective des secondes nervures (60) lorsque la pince de ligature (10) est en position pincée, l'angle β étant compris entre environ 75 degrés et environ 85 degrés.

2. Pince de ligature (10) selon la revendication 1, dans laquelle l'angle β vaut d'environ 80 degrés.

3. Pince de ligature (10) selon l'une quelconque des revendications précédentes, dans laquelle les première et seconde mâchoires (12, 14) soutiennent des bossages (32, 42), les bossages (32, 42) étant conçus pour entrer en prise avec un applicateur de pince.

4. Pince de ligature (10) selon l'une quelconque des revendications précédentes, dans laquelle la pince de ligature (10) est formée d'un matériau polymère.

5. Pince de ligature (10) selon l'une quelconque des revendications précédentes, dans laquelle la première mâchoire (12) comporte un premier élément de verrouillage (30) et dans laquelle la seconde mâchoire (14) comporte un second élément de verrouillage (40), le second élément de verrouillage (40) pouvant pivoter en prise avec le premier élément de verrouillage (30) afin de retenir la pince de ligature (10) en position pincée.

6. Pince de ligature (10) selon l'une quelconque des revendications précédentes, dans laquelle les première et seconde mâchoires (12, 14) sont incurvées le long de l'axe longitudinal de la pince de ligature (10).

7. Pince de ligature (10) selon l'une quelconque des revendications précédentes, dans laquelle la seconde mâchoire (14) soutient des éléments de prise avec du tissu (44) qui sont conçus pour saisir et pour étirer du tissu lorsque la pince de ligature (10) est déplacée de la position ouverte vers la position pincée.

8. Pince de ligature (10) selon la revendication 7, dans laquelle chacun des éléments en prise avec du tissu (44) comporte une face distale (44a) inclinée vers le bas dans une direction distale.

9. Pince de ligature (10) selon l'une quelconque des revendications précédentes, dans laquelle la première mâchoire (12) est accouplée de manière pivotante à la seconde mâchoire (14) par une charnière vivante.

10. Pince de ligature (10) selon l'une quelconque des revendications précédentes, dans laquelle les nervures (60) présentent une configuration rectangulaire.

11. Pince de ligature (10) selon l'une quelconque des revendications précédentes, dans laquelle les parois (62) des premières nervures (60) sont en opposition avec la seconde surface de pincement (28) de la seconde mâchoire (14) et où les parois (62) des secondes nervures (60) en opposition à la première surface de pincement (22) de la première mâchoire (12) sont plates ; ou
dans laquelle les parois (62) des premières nervures (60) en opposition à la seconde surface de pincement (28) de la seconde mâchoire (14) et les parois (62) des secondes nervures (60) en opposition à la première surface de pincement (22) de la première mâchoire (12) sont incurvées.

12. Pince de ligature (10) selon la revendication 5, dans laquelle la seconde mâchoire (14) comporte un élément de retenue élastique (45), l'élément de retenue élastique (45) étant positionné pour être mis en prise par le premier élément de verrouillage (30) de la première mâchoire (12) lorsque la pince de ligature (10) est déplacée jusqu'à la position pincée, afin de retenir la pince de ligature (10) en position pincée.
